# EUROPEAN PATENT APPLICATION

(11) **EP 4 491 201 A1**
(43) Date of publication of application: **15.01.2025**
(21) Application number: 24150183.2
(22) Date of filing: 03.01.2024
(51) Int. Cl.: A61L 15/60, C08F 220/06, C08F 289/00, A61F 13/15

(54) **SUPERABSORBENT POLYMER COMPOSITION, COMPOSITE LAYERED STRUCTURE INCLUDING THE SAME AND METHOD OF MANUFACTURING SUPERABSORBENT POLYMER**

(30) Priority: 12.07.2023 TW 112126003
(71) Applicant: Formosa Plastics Corporation, Kaohsiung City (TW)
(72) Inventor: CHEN, Zhong-Yi, Kaohsiung City (TW); HUANG, Li-Han, Kaohsiung City (TW); CHENG, Ping-Chun, Kaohsiung City (TW)
(74) Representative: Lang, Christian

(57) **Abstract**

The present invention is related to a superabsorbent polymer composition, a composite layered structure including the same and a method of manufacturing a superabsorbent polymer. The superabsorbent polymer composition includes a water solution containing carboxylic acid monomers, a hydrolyzed collagen, a crosslinking agent, and a polymerization initiator. The superabsorbent polymer composition includes a specific amount and a specific molecular weight of hydrolyzed collagen, and thus the obtained superabsorbent polymer has high absorbent properties and dryness properties as well as proper biodegradability.

## Description

### BACKGROUND

### Field of Invention

The present invention relates to a superabsorbent polymer composition. More particularly, the present invention relates to a superabsorbent polymer composition including hydrolyzed collagen and having high absorbent properties, a high dryness properties, and proper biodegradability.

### Description of Related Art

Superabsorbent polymer is a polymer material that can absorb and retain liquid, and the mass of liquid absorbed by the superabsorbent polymer can be up to 50 times to 500 times the weight of the superabsorbent polymer or more. Since it has excellent absorbent properties, the superabsorbent polymer can not only be widely used as personal sanitary products (e.g. diapers, feminine hygiene products, disposable wipes, and/or incontinence underwear) but also be widely applied to agricultural or horticultural industries (such as water-retaining agents), building industry (such as an antidew condensation agent), petroleum industry (such as a dehydrating agent), cable line manufacturing industry (such as a waterproof cladding agent), pharmaceutical industry (such as a wound dressing) as well as entertainment industry (such as an expandable water toy and artificial snow), etc.

The superabsorbent polymer is widely used but thus causes waste problems. The conventional treatment methods are burning treatment or landfilling treatment. However, burning treatment will cause further problems such as air pollution or carbon emission. The landfilling treatment is limited by the low efficiency of biological degradation of superabsorbent polymer and will still lead to environmental burdens.

Therefore, superabsorbent polymer having biodegradability has become a new research hotspot. Among them, Japan Patent Publication No. 1999-343339 discloses a superabsorbent polymer produced by the reaction of a poly-γ-glutamate with a polyepoxy compound. U.S. Patent Publication No. 2017/0002098 discloses to use polysaccharide hydrogels. China Patent Publication Nos. 1410463 and 1846544 respectively disclose the use of biomaterials such as Konjac powder and Yacon as crosslinking agents. Taiwan Patent No. I432501B discloses the addition of unsaturated double-bond compounds or epoxy compounds for polymerizing reactions.

Although the abovementioned methods help remove the wastes of superabsorbent polymer; however, polysaccharide hydrogels not only have disadvantages such as complicated processing processes but also obtain superabsorbent polymer with problems such as being too soft or having poor performance. Also, superabsorbent polymer cannot provide some functions required by modern sanitary products. Poly-γ-glutamate needs to be produced by microorganism culture and thus is required to be sterilized before use, resulting in not only increased production cost but also a more complicated processing process. Although the superabsorbent polymer using starch as a copolymer has high biodegradability, starch may become yellow during a heating process and result in negative experience to consumers. By adding unsaturated double-bond compounds or epoxy compounds for polymerizing reactions, the biodegradability of such superabsorbent polymer can be increased though; the reliance on petrochemical raw materials can be further decreased if it contains natural materials.

In view of these, it is necessary to have a superabsorbent polymer having high absorbent properties, high dryness properties, and proper biodegradability to solve the aforementioned problems.

### SUMMARY

Therefore, an aspect of the present invention is to provide a superabsorbent polymer composition including hydrolyzed collagen.

Another aspect of the present invention is to provide a method for manufacturing a superabsorbent polymer. The method includes adding hydrolyzed collagen so that the obtained superabsorbent polymer has high absorbent properties, high dryness properties, and proper biodegradability.

According to the abovementioned aspect of the present invention, a superabsorbent polymer composition is provided, in which the superabsorbent polymer composition includes a water solution containing carboxylic acid monomers, hydrolyzed collagen, a crosslinking agent, and a polymerization initiator. The neutralization ratio of the water solution containing the carboxylic acid monomers is bigger than or equal to 45 mole percentage and the pH value of the water solution containing the carboxylic acid monomers is smaller than or equal to 7.0. Moreover, the aforementioned polymerization initiator includes a thermal decomposition initiator and/or a redox initiator.

According to an embodiment of the present invention, a molecular weight of the hydrolyzed collagen is bigger than or equal to 1 KDa and smaller than 10 KDa.

According to an embodiment of the present invention, based on superabsorbent polymer composition as 100 weight percent, an amount of the hydrolyzed collagen is 0.1 weight percent to 20 weight percent.

According to an embodiment of the present invention, the superabsorbent polymer composition can selectively include a surface treatment agent.

According to an embodiment of the present invention, the hydrolyzed collagen is obtained by subjecting fish scales to a hydrolyzing step with a protease.

According to an embodiment of the present invention, the protease is serine endopeptidase.

According to another aspect of the present invention, a method for manufacturing a superabsorbent polymer is provided. Initially, a neutralizing step is performed on a water solution of carboxylic acid monomers by using a basic compound, so as to obtain a water solution containing carboxylic acid monomers. The neutralization ratio of the water solution containing the carboxylic acid monomers is bigger than or equal to 45 mole percentage, and a pH value of the water solution containing the carboxylic acid monomers is smaller than or equal to 7.0.

Then, the water solution containing the carboxylic acid monomers, hydrolyzed collagen, a crosslinking agent and a polymerization initiator are mixed to perform a free radical polymerization reaction. The hydrolyzed collagen is made by subjecting fish scales to a hydrolyzing step with a protease. Further, a drying step is performed after performing the free radical polymerization reaction to obtain the superabsorbent polymer.

According to an embodiment of the present invention, the method for manufacturing the superabsorbent polymer can selectively include performing a surface cross-linking treatment on the superabsorbent polymer.

According to an embodiment of the present invention, the protease is serine endopeptidase.

According to an embodiment of the present invention, a molecular weight of the hydrolyzed collagen is bigger than or equal to 1 KDa and smaller than 10 KDa.

With the application of the superabsorbent polymer composition and the method for manufacturing the superabsorbent polymer, in which the superabsorbent polymer composition includes hydrolyzed collagen, the obtained superabsorbent polymer can have high absorbent properties, high dryness properties, and proper biodegradability.

### DETAILED DESCRIPTION

As described in the above, the present invention provides a superabsorbent polymer composition including hydrolyzed collagen, and the obtained superabsorbent polymer has high absorbent properties, high dryness properties, and proper biodegradability, which can obtain an absorbent by further mixed with a fiber substrate.

The term "absorbent properties" herein refers to the ability of the superabsorbent polymer to absorb liquid. The liquid can be pure water or a water solution. In some specific embodiments, the water solution can include but not limited to saline, urine, menstrual blood, soil water, or other water solution applied in the environment. It is worth noting that salt may affect the absorbent properties of the superabsorbent polymer since the superabsorbent polymer can absorb a water solution containing salt having a weight less than that of pure water. To simulate the real situation, the absorbent properties of the superabsorbent polymer can be evaluated by using saline or synthetic urine.

In some embodiments, the absorbent properties can be evaluated by absorption rate (also referred to as free swell capacity, FSC), which is the ratio value of the weight of liquid absorbed by a superabsorbent polymer while the superabsorbent polymer is not under external force to the dry weight of the superabsorbent polymer not absorbing the liquid.

In some other embodiments, the absorbent properties can be evaluated while the superabsorbent polymer is under external force. In some specific embodiments, the absorbent properties can be evaluated by a centrifuge retention capacity (CRC), which is the ratio value of the weight of liquid retaining in the superabsorbent polymer after the superabsorbent polymer absorbs the liquid and is centrifuged to the dry weight of the superabsorbent polymer not absorbing the liquid.

In some specific embodiments, the absorbent properties can be evaluated by absorption against pressure (AAP), which is the ratio value of the weight of liquid retaining in the superabsorbent polymer after the superabsorbent polymer absorbs the liquid for a while to the dry weight of the superabsorbent polymer not absorbing the liquid.

In some specific embodiments, the absorbent properties can be evaluated by an AAP index (i.e., index under the absorption against pressure), which is the ratio value of CS AAP (core-shell absorption against pressure) to AAP. The method to evaluate CS AAP is the same as that of the AAP index, while the difference is that the absorbent time is longer for evaluating the CS AAP.

The term "high absorbent properties" herein refers to a superabsorbent polymer having an absorption rate bigger than or equal to 50 g/g, an AAP bigger than or equal to 15 g/g, preferably bigger than or equal to 20 g/g, and more preferably bigger than or equal to 30 g/g, and an AAP index of bigger than or equal to 0.8. As proven by experiments, the superabsorbent polymer of the present invention has an absorption rate of 50 g/g to 65 g/g, an AAP of 20 g/g to 30 g/g, and an AAP index of bigger than or equal to 0.8 and smaller than 0.9.

The term "dryness properties" herein refers to the degree of difficulty in releasing liquid after a superabsorbent polymer absorbs liquid. In some embodiments, the dryness properties can be evaluated by a rewet amount of the absorbent made by the superabsorbent polymer. The rewet amount is the amount of liquid released by an absorbent under pressure after the absorbent absorbs the liquid. The term "high dryness properties" herein refers to a rewet amount smaller than 5 g. As proven by experiments, the absorbent obtained by the superabsorbent polymer of the present invention has a rewet amount smaller than 5 g, e.g., 0.1 g to 2.0 g, or 0.5 g to 1.5 g.

The term "biodegradability" herein refers to the degree of difficulty for microorganisms to degrade the superabsorbent polymer. In some embodiments, biodegradability can be evaluated by the gel strength of a superabsorbent polymer after the superabsorbent polymer is treated with L-ascorbic acid. It is worth noting that L-ascorbic acid and the salt of the same can make the superabsorbent polymer swell and deteriorate, and therefore help the organisms to degrade the superabsorbent polymer. However, L-ascorbic acid and the salt of the same also exist in liquids such as urine, menstrual blood, and soil water. The biodegradability should be controlled in a proper range since the durability of the obtained absorbent is worse when the gel strength is too low after the superabsorbent polymer is treated by L-ascorbic.

The term "suitable biodegradability" herein refers to that the gel strength of a superabsorbent polymer treated by L-ascorbic for 3 hours is 100 g to 200 g, and the gel strength of the superabsorbent polymer treated by L-ascorbic for 24 hours is 50 g to 110 g. As proved by experiments, the superabsorbent polymer of the present invention treated by L-ascorbic for 3 hours has a gel strength of 100 g to 150 g, or 110 g to 135 g. Moreover, the superabsorbent polymer of the present invention treated by L-ascorbic for 24 hours has a gel strength of 42 g to 80 g, or 50 g to 75 g.

Hydrolyzed collagen is added in the present invention so that the superabsorbent polymer has high absorbent properties, high dryness properties, and proper biodegradability. In detail, the superabsorbent polymer composition includes a water solution containing carboxylic acid monomers, hydrolyzed collagen, a crosslinking agent, and a polymerization initiator.

The water solution containing the carboxylic acid monomers is obtained by dissolving a carboxylic acid monomer composition in water, followed by a neutralizing step with a basic compound. The carboxylic acid monomer composition can include but not limited to acrylic acid composition, other suitable carboxylic acid monomer composition, or any mixtures of the aforementioned compositions. In some specific embodiments, the acrylic acid composition can include but not limited to acrylic acid, methacrylic acid, 2-carboxylic acid ethyl ester, 2-carboxylic acid ethyl methacrylate, methyl acrylate and/or ethyl acrylate. In some embodiments, the carboxylic acid monomer composition can selectively include water-soluble monomers with other acid groups and unsaturated double bonds. The water-soluble monomers can include but not limited to 2-propenylamine-2-methylpropanesulfonic acid, maleic acid, fumaric acid, and/or other appropriate water-soluble monomers and/or other suitable water-soluble monomers. In other embodiments, other hydrophilic monomers having unsaturated double bonds can be selectively added to the water solution containing the carboxylic acid monomers. The hydrophilic monomers having unsaturated double bonds can be maleic acid, fumaric acid, acrylamide, methacrylamide, dimethylaminopropyl acrylamide, (acrylamidopropyl)trimethylammonium chloride, for example.

The concentration of the water solution of the carboxylic acid monomers can be 20 weight percent to 55 weight percent, or 30 weight percent to 45 weight percent, for example. The water solution of the carboxylic acid monomers having a concentration in the range can be prepared more easily, and the reaction heat of the subsequent free radical reaction can be controlled more easily. Moreover, the obtained superabsorbent polymer can have better mechanical properties and benefits of the mechanical process.

The neutralizing step performed on the water solution of the carboxylic acid monomers can be performed with a basic compound. In some embodiments, the basic compound can include but not limited to an alkali metal compound, an alkaline earth metal compound, other suitable basic compounds, or any mixtures of the aforementioned compounds. In some embodiments, the basic compound can include a basic compound having a hydroxyl group, a compound having a carboxyl group, other suitable basic compounds, or any mixtures of the aforementioned materials. In some specific embodiments, the basic compound can include but not limited to sodium hydroxide, potassium hydroxide, sodium carbonate, potassium carbonate, and/or an ammonia compound.

As the neutralizing step is performed, the basic compound is slowly added to the water solution of the carboxylic acid monomers. Calculated by volume, the ratio of the basic compound and the monomers with a carboxylic acid group can be 0.80 to smaller than 1.00, or 0.85 to 0.95, for example, and the time for performing the neutralizing step can be 1 hour to 3 hours to control the temperature of the water solution containing the carboxylic acid monomers at 15°C to 40°C.

After the neutralizing step, the partial carboxylic acid of the obtained water solution containing the carboxylic acid monomers can be formed into sodium salts, potassium salts, or ammonium salts. Therefore, the dissociation of the hydrophilic group is affected, and the absorbent properties of the obtained superabsorbent polymer can be improved. However, when the water solution containing the carboxylic acid monomers has a lower amount of carboxylic acid, the structure of the obtained superabsorbent polymer will in turn be destroyed, and the absorbent properties worsen. Thus, the amount of carboxylic acid in the water solution containing the carboxylic acid monomers should be controlled in the neutralizing step.

The amount of carboxylic acid group in the water solution containing the carboxylic acid monomers can be evaluated by the neutralization ratio. The neutralization ratio is the amount of the carboxylic acid group being neutralized in the water solution containing the carboxylic acid monomers. Based on the amount of carboxylic acid of the water solution containing the carboxylic acid monomers not subjected to the neutralizing step as 100 mole percentage, the neutralization ratio of the water solution containing the carboxylic acid monomers can be bigger than or equal to 45 mole percentage, e.g., 45 mole percentage to 85 mole percentage, or 50 mole percentage to 75 mole percentage.

The amount of carboxylic acid in the water solution containing the carboxylic acid monomers can also be evaluated by pH value, which the pH value of the carboxylic acid monomers is smaller than or equal to 7.0, e.g., 5.0 to 7.0, or 5.5 to 6.5. When the pH value of the carboxylic acid monomers is bigger than 7.0, the obtained superabsorbent polymer has defects such as more amounts of residual monomers and decreased absorbent properties. Moreover, the problems such as causing skin allergies easily after attaching the skin may also occur. When the pH value of the water solution containing the carboxylic acid monomers is 5.0 to 7.0, the obtained superabsorbent polymer has a lower amount of residual monomers and better absorbent properties.

The water solution containing the carboxylic acid monomers can selectively include a water-soluble polymer. The molecular weight of the water-soluble polymer is not specially limited, and, understandably, that the molecular weight of the added water-soluble polymer will not affect the absorbent properties of the obtained superabsorbent polymer. In some embodiments, the water-soluble polymer can include but not limited to partially saponified or fully saponified polyvinyl alcohol, polyethylene glycol, polyacrylic acid, polyacrylamide, starch and/or starch derivatives such as methylcellulose, methylcellulose acrylate and/or ethyl cellulose. In some specific embodiments, the water-soluble polymer can be starch and/or partially saponified or fully saponified polyvinyl alcohol.

Based on the amount of the carboxylic acid monomers as 100 weight percent, the amount of water-soluble polymer can be 0 weight percent to 20 weight percent, e.g., bigger than 0 weight percent to 10 weight percent, or bigger than 0 weight percent to 5 weight percent, so that the obtained superabsorbent polymer has a better absorbent properties.

The hydrolyzed collagen is obtained by subjecting the collagen to a hydrolyzing step. The source of collagen is not specially limited and can originate from the skin, the bone, or the cartilage of land animals such as a cow, a pig, or poultry, or the skin, the bone, the fin, the scale, and/or the swim bladder of aquatic animals. In some specific embodiments, the collagen can be originated from the fish scales of milkfish.

The hydrolyzing step can include but not limited to acidic hydrolysis, alkaline hydrolysis, and/or enzyme hydrolysis. The enzyme hydrolysis can be referred to the review paper "Biological activity of peptides purified from fish skin hydrolysates" published by Racheal Abuine et al. in volume 22, No. 10 of Fisheries and Aquatic Sciences in 2019, the article "Studies on Antioxidative Activities of Hydrolysates from Fish Scales Collagen of Tilapia" published by Chih-Hsing Lai et al. in volume 15, issue 2, pages 99-108 of Journal of Taiwan Fisheries Research in 2007, and Taiwan Patent Application No. 200831005A, which are list herein as reference. In some embodiments, the hydrolyzed collagen can be the commercially available product, e.g., the fish scales hydrolyzed collagen (product name: FCMB101) manufactured by Nuowant Biomedical Technology Co., Ltd.

The kinds of protease are not specially limited. In some embodiments, from the perspective of whether the protease reacts at the end of collagen, the protease can be an endoprotease. In some embodiments, from the perspective of what the amino acid is in the active site where the protease reacts, the protease can include but not limited to serine proteases, threonine proteases, cysteine proteases, aspartic proteases, glutamic acid proteases, and/or metalloproteases. In some specific embodiments, the protease can be alcalase, papain and/or bromelain. It is noted that the alkaline proteolytic enzyme can be the protease of *Bacillus licheniformis,* which is one of the serine endopeptidases.

Based on the amount of the carboxylic acid monomers as 100 weight percent, the amount of hydrolyzed collagen can be 0.1 weight percent to 20 weight percent, e.g., 0.5 weight percent to 10 weight percent, or 0.5 weight percent to 5 weight percent. When the amount of hydrolyzed collagen is in the aforementioned range, the obtained superabsorbent polymer has high dryness properties and proper biodegradability.

The molecular weight of hydrolyzed collagen can be bigger than or equal to 1 KDa and smaller than 10 KDa, for example, e.g., bigger than or equal to 1 KDa and smaller than or equal to 5 KDa. When the molecular weight of hydrolyzed collagen is in the aforementioned range, the obtained superabsorbent polymer can have high absorbent properties, high dryness properties, and proper biodegradability.

After mixing the water solution containing the carboxylic acid monomers and the hydrolyzed collagen, the crosslinking agent and the polymerization initiator are added to perform a free radical polymerization reaction. The aforementioned free radical polymerization reaction can be performed in a batch reactor or a conveyor belt reactor. After the free radical polymerization reaction, the hydrolyzed collagen can physically disperse in the cross-linking and bridging structure.

As the free radical polymerization reaction is performed, the crosslinking agent renders the obtained superabsorbent polymer to have a suitable degree of cross-linking to form a hydrogel having appropriate processability. The kind of crosslinking agent is not limited and can include a compound with at least two unsaturated double bond groups, a compound with at least two epoxy groups, other suitable crosslinking agents, or any mixtures of the aforementioned compounds. In some specific embodiments, the compound with at least to unsaturated double bond groups can include but not limited to N,N'-bis(2-propenyl) amine, N,N'-methylene-bis(acrylamide), N,N'-methylenebis(methacrylamide), allyl acrylate, ethylene glycol diacrylate, polyethylene glycol diacrylate, ethylene glycol dimethacrylate, poly ethylene glycol dimethacrylate, glycerol triacrylate, glycerol trimethacrylate, glycerol ethylene oxide triacrylate, glycerol ethylene oxide trimethacrylate, trimethylolpropane ethylene oxide triacrylate, trimethylolpropane ethylene oxide trimethacrylate, trimethylolpropane trimethacrylate, trimethylolpropane triacrylate, N,N,N-tris(2-propenyl)amine, ethyleneglycol diacrylate, polyoxyethylene triacrylate glycerol esters, triethylene polyoxyethylene glycerol triacrylate, and/or dipropylene triethylene glycol esters. In some specific embodiments, the compound with at least two unsaturated epoxy groups can include but not limited to sorbitol polyglycidyl ether, polypropylene glycol polyglycidyl ether, ethylene glycol diglycidyl ether, diethylene glycol diglycidyl alcohol ether, polyethylene glycol diglycidyl ether, and/or dipropylene glycol polyglycidyl ether.

Based on the amount of the solid content of superabsorbent polymer composition as 100 parts by weight, the amount of the crosslinking agent can be 0.001 parts by weight to 5 parts by weight, e.g., 0.01 parts by weight to 3 parts by weight. When the amount of crosslinking agent is in the aforementioned range, the formed hydrogel has better mechanical properties and benefits mechanical processing.

The polymerization initiator can decompose and generate free radicals to initiate the aforementioned free radical polymerization reaction, thereby forming the hydrogel. The polymerization initiator of the present invention can include but not limited to a thermal decomposition initiator, a redox initiator, other suitable polymerization initiator, or any mixtures of the aforementioned materials. In some specific embodiments, the redox initiator can include an acidic sulfite salt, a thiosulfate salt, ascorbic acid or a ferrous salt, and/or ammonium persulfate. In some specific embodiments, the thermal decomposition initiator can include hydrogen peroxide (i.e., hydrogen peroxide), di-t-butyl peroxide, a peroxyamide, a persulfate (such as ammonium salt, alkali metal salt), and/or azo compound (e.g., 2,2'-azobis (2-amidinopropane) dihydrochloride salt, 2.2'-azobis (N,N-dimethyleneisobutylamidine) dihydrochloride salt).

When the polymerization initiator includes a thermal decomposition initiator and a redox initiator at the same time, the redox initiator can decompose and generate free radicals, thereby initiating the first stage of free radical polymerization reaction. At the same time, the reaction heat generated in the first stage of the free radical polymerization reaction can make the thermal decomposition initiator decompose, thereby increasing the reactivity of the free radical polymerization reaction.

Based on the amount of the carboxylic acid monomer composition being neutralized as 100 weight percent, the amount of polymerization initiator is 0.001 weight percent to 10 weight percent, e.g., 0.1 weight percent to 5 weight percent. When the amount of the polymerization initiator is 0.001 weight percent to 10 weight percent, the free radical polymerization reaction can have a suitable reactivity and can prevent the obtained superabsorbent polymer from forming a solid but not a hydrogel due to the degree of polymerization that is too high.

After the free radical polymerization reaction is performed, the drying step is further performed to obtain a superabsorbent polymer. By performing the drying step, the water content and residual monomers in the hydrogel can be removed effectively. Therefore, compared to the superabsorbent polymer not subjected to the drying step, the superabsorbent polymer obtained after being subjected to the drying step can have better absorbent properties. In some embodiments, the temperature of the drying step can be 100°C to 180°C, for example, and the temperature of the drying step can be 1 hour to 3 hours, for example, to remove the water content and the residual monomers in the superabsorbent polymer effectively as well as to control the degree of crosslinking of the superabsorbent polymer.

Before the drying step is performed, a pulverizing step can be performed on the hydrogel to obtain hydrogel pieces. In some embodiments, the pulverizing step can be performed by using a cutting mill. The diameters of the hydrogel pieces can be smaller than or equal to 2.0 mm, for example, or 0.03 mm to 1.5 mm. When having diameters in the aforementioned range, the hydrogel pieces have better thermal conductivity properties and higher amounts of fine powders. Thus, it is beneficial to remove residual monomers and water content and prevent the cost of the subsequent processing from increasing. It is worth noting that the narrower the distributions of the diameters of the hydrogel pieces are, the better the efficiency of the drying step is, and the absorbent properties of the formed dry matter will be better.

After performing the drying step, the superabsorbent polymer is further pulverized and screened to obtain the powder of the superabsorbent polymer, which the diameter of the powder of the superabsorbent polymer can be 0.06 mm to 1.00 mm, or 0.10 mm to 0.85 mm, for example, to achieve a better amount of fine powder and absorbent properties.

After the free radical polymerization reaction and the drying step, the obtained superabsorbent polymer has an even cross-linking structure. Thus, the superabsorbent polymer is an un-dissolved hydrophilic polymer. To further increase the absorption rate of the superabsorbent polymer, the water-absorbing properties such as the gel strength, the anti-blocking properties, and the liquid permeability, the surface cross-linking treatment can be selectively performed on the superabsorbent polymer, so that the surface of the superabsorbent polymer can be further bridged. The surface cross-linking treatment includes a mixture of the surface treatment agent and the superabsorbent polymer and is subjected to a heat treatment at 140°C to 160°C for 0.5 hours to 1.5 hours.

The surface treatment agent for the surface cross-linking treatment can include but not limited to a multifunctional crosslinking agent that can react to carboxylic acid reaction. In some embodiments, the surface treatment agent can include but not limited to a polyhydric alcohol, a polyamine, a compound having at least two epoxy groups, an alkylene carbonate, other suitable crosslinking agent, or any mixtures of the aforementioned materials. In some specific embodiments, the surface treatment agent can include but not limited to glycerol, ethylene glycol, diethylene glycol, triethylene glycol, polyethylene glycol, propylene glycol, 1,4-butanediol, ethylenediamine, di ethylenediamine, triethylenediamine, sorbitol polyglycidyl ether, polypropylene glycol polyglycidyl ether, ethylene glycol diglycidyl ether, diethylene glycol diglycidyl ether, polyethylene glycol diglycidyl ether, dipropylene glycol polyglycidyl ether, ethylene glycol carbonate, 4-methyl-1,3-dioxolan-2-one, 4,5-dimethyl-1,3-dioxolan-2-one, 4,4-dimethyl-1,3-dioxolan-2-one, 4-ethyl-1,3-dioxolan-2-one, 1,3-dioxan-2-one or 4,6-dimethyl-1,3-dioxan-2-one and/or 1,3-dioxepan-2-one.

Based on the amount of the superabsorbent polymer composition as 100 parts by weight, the amount of surface treatment agent can be 0.001 parts by weight to 10 parts by weight, for example, preferably 0.005 parts by weight to 5 parts by weight, so that the suitable cross-linking effect can be achieved, and the absorbent properties of the obtained superabsorbent polymer can be increases.

According to the form of the surface treatment agent, the surface treatment agent can added to the superabsorbent polymer composition directly or after being prepared into a cross-linking solution at first. The cross-linking solution can be water or a hydrophilic organic solvent (e.g., methanol, ethanol, propanol, isobutanol, acetone, methyl ether, and/or ethyl ether). In some embodiments, the hydrophilic organic solvent is preferably methanol or ethanol.

In some specific embodiments, the obtained superabsorbent polymer of the present invention can be applied in hygiene supplies such as diapers (e.g., a low-consistency pulp (Fluffless, which uses a lot of superabsorbent polymer at the same time) or incontinence underwear. Therefore, the diaper has high absorbent properties, high dryness properties, and proper biodegradability.

In some application examples, the superabsorbent polymer can be used to make an absorbent, which the absorbent can be obtained by mixing the superabsorbent polymer and a fiber substrate. In general, the basis weight (weight per unit area) of the absorbent can be 0.01 g/cm² to 0.30 g/cm², and the thickness of the absorbent is not greater than 30 mm.

In some embodiments, the fiber substrate can be a hydrophilic fiber that benefits the diffusion and transmission of liquid, thereby increasing the absorbent properties of absorbent. In some specific embodiments, the hydrophilic fiber can include but not limited to pulverized wood pulp.

In some implements, based on the weight of the absorbent as 100 weight percent, the weight of the superabsorbent polymer is bigger than or equal to 20 weight percent and smaller than 100 weight percent, e.g., bigger than or equal to 40 weight percent and smaller than 100 weight percent, or bigger than or equal to 50 weight percent and smaller than 100 weight percent.

In practical application, the absorbent can be disposed on a non-permeable polyethylene membrane. Moreover, a permeable nonwoven fabric is deposed on the absorbent to form a composite layered structure. According to the requirement of the supplication, in other application examples, the composite layered structure can include no fiber substrates. In other words, the superabsorbent polymer can be deposited on the polyethylene membrane directly, and the nonwoven fabric is deposited on the superabsorbent polymer. In these practical applications, the superabsorbent polymer paved between the polyethylene membrane and the nonwoven fabric is not tightly stacked, so that the superabsorbent polymer has spaces for expansion and can absorb water more efficiently.

### Preparation Example 1

An acrylic acid water solution was obtained by mixing 540 g of acrylic acid and 583 g of water. Then, 437.5 g of a sodium hydroxide water solution with a concentration of 48 weight percent was added to the acrylic acid water solution to perform a neutralizing step, which the ratio of sodium hydroxide to acrylic acid was 0.85 to 0.95, the time of the neutralizing step was 2 hour, and in the process of the neutralizing step, the temperature of the acrylic acid water solution was 15°C to 40°C. After the neutralizing step, the acrylic acid water solution with a monomer concentration of 42 weight percent could be obtained, which 70 mole percentage of acrylic acid was neutralized to sodium acrylate, and the acrylic acid water solution was pH 5.71.

Next, the acrylic acid water solution and 15.60 g of the hydrolyzed collagen (Product name: FCMB101; manufacture: Nuowant Biomedical Technology Co., Ltd) having a molecular weight of 1500 g/mol were mixed, followed by the addition of 0.9 g of methylene-bis(acrylamide). The temperature was kept at about 20°C. Then, 3.6 g of sodium bisulfite, 3.6 g of ammonium persulfate, and 0.3 g of hydrogen peroxide were added to perform a free radical polymerization reaction, thereby obtaining a hydrogel. A cutting mill was used to chop hydrogel into hydrogel pieces having particle sizes smaller than or equal to 2.0 mm.

Next, a drying step was performed on the hydrogel pieces at 130°C for 2 hours, followed by a screening using a sifting screen of 0.10 mm to 0.85 mm, thereby obtaining powders. Then, 200 g of the powders were added into 5 g of a surface treatment agent and an organic solvent, followed by a heat treatment at 150°C for 1 hour. After standing till cooling down, the superabsorbent polymer of Preparation Example 1 was obtained. The surface treatment agent solution included ethylene glycol, 1,4-butanediol (manufactured by FORMOSA PLASTICS CORPORATION), the organic solvent included methanol, and the volume ratio of ethylene glycol, 1,4-butanediol and methanol was 1:1:0.5.

### Preparation Example 2 to Preparation Example 5, Comparative Preparation

### Example 1 to Comparative Preparation Example 3

The methods for manufacturing superabsorbent polymers of Preparation Example 2 to Preparation Example 5 and Comparative Preparation Example 1 to Comparative Preparation Example 3 were the same as that of Preparation Example 1, but there were differences in the amount of the hydrolyzed collagen, the molecular weight, and/or and the methods for preparation. In details, compared to the hydrolyzed collagen of Preparation Example 1, 15.60 g of the hydrolyzed collagen (product name: FCMB101, which the molecular weight was 3000 g/mol) were used for Preparation Example 2, the amount of the hydrolyzed collagen (product name: FCMB101) of Preparation Example 3 was increased to 46.80 g, and the amount of the hydrolyzed collagen (product name: FCMB101) of Preparation Example 4 was decreased to 7.80 g.

The hydrolyzed collagen added in the superabsorbent polymer of Preparation Example 5 was obtained by subjecting the collagen of fish scales to a hydrolyzing step with commercial alcalase, and the amount of the hydrolyzed collagen was 15.6 g. The hydrolyzing step was referred to the article "Studies on Antioxidative Activities of Hydrolysates from Fish Scales Collagen of Tilapia" published by Chih-Hsing Lai et al. on volume 15, issue 2, pages 99-108 of Journal of Taiwan Fisheries Research in 2007, which was briefly described in the followings: after the acid deliming treatment was performed on the fish scales, the protease was added to perform a hydrolyzing step at 45°C to 55°C for 1 hour to 5 hours, thereby obtaining hydrolyzed collagen.

The superabsorbent polymer of the Comparative Preparation Example 1 had no hydrolyzed collagen added; the amount of the hydrolyzed collagen (product name: FCMB101) added to the superabsorbent polymer of the Comparative Preparation Example 2 was reduced to 0.83 g; the hydrolyzed collagen of the superabsorbent polymer of Comparative Preparation Example 3 was obtained with the method same as that of Preparation Example 5, but the molecular weight was 10000 g/mol (i.e., 10 KDa).

It was worth nothing that the molecular weight of the hydrolyzed collagen could be detected by using a liquid chromatograph, which the column was Superdex 30 increase column, the eluant included 0.1 M sodium sulfate (Na₂SO₄), 0.02 M, pH 5.3 sodium dihydrogen phosphate (NaH₂PO₄), and 0.5% sodium dodecyl sulfate (SDS), while the flow rate of the eluant was 0.5 mL/minute.

The amounts and the molecular weights of the hydrolyzed collagen of Preparation Example 1 to Preparation Example 5, Comparative Preparation Example 1 to Comparative Preparation Example 3 were recorded in Table 1.

### Preparation Example 6

A mixture was prepared by mixing 10.0 g of the superabsorbent polymer of Preparation Example 1 and 10.0 g of pulverized wood pulp with an absorbentmaking machine. Then, the metal mesh of 400 mesh (i.e., an aperture of 38 µm) was used to shape the mixture, thereby obtaining an absorbent having an area of 160 cm² (8 cm×20 cm). Then, the shaped absorbent was placed on the (polyethylene, PE) membrane and the nonwoven fabric was covered on the absorbent. Hereafter, a pressure (with an area of 160 cm² and weight of 30 kg) of 18.39 kPa was exerted on the absorbent, and after 5 minutes, the peripheries of the absorbent, the PE membrane, and the nonwoven fabric were glued with a white glue, thereby obtaining the testing absorbent of Preparation Example 6.

### Preparation Example 7 to Preparation Example 10 and Comparative Preparation Example 4 to Comparative Preparation Example 6

The manufacturing methods of the testing absorbent of Preparation Example 7 to Preparation Example 10 and Comparative Preparation Example 4 to Comparative Preparation Example 6 were similar to that of Preparation Example 6. The differences were in that the superabsorbent polymers of Preparation Example 2 to Preparation Example 5 and Comparative Preparation Example 1 to Comparative Preparation Example 3 were used in Preparation Example 7 to Preparation Example 10 and Comparative Preparation Example 4 to Comparative Preparation Example 6, respectively.

### Evaluation method

The absorption rate, the centrifuge retention capacities, the AAP, the CS AAP, the AAP indices, the residual monomers, and the gel strengths of Preparation Example 1 to Preparation Example 7, Comparative Preparation Example 1 to Comparative Preparation Example 3 were evaluated using the methods described in the followings, and the results were recorded in Table 1.

Moreover, the rewet amounts of the obtained test absorbents of Preparation Example 6 to Preparation Example 10 and Comparative Preparation Example 4 to Comparative Preparation Example 6 were evaluated using the evaluating methods described in the followings, and the results were recorded in Table 2. It was worth noting that unless otherwise specified, the evaluating methods were performed at a temperature of 23±2°C and a relative humidity of 45±10% in the air. Before performing the evaluating methods, the superabsorbent polymers should be mixed thoroughly.

### Retention capacity

The retention capacity was determined by the test methods of ERT 441.3(10) by European Disposables and Nonwovens Association (EDANA).

### AAP

The AAP of the superabsorbent polymer was determined by 442.3(10) by EDANA and would be briefly described as follows: The initial weight of the superabsorbent polymer was measured. Then a pressure of 4.9 kPa was exerted on the superabsorbent polymer, and after the superabsorbent polymer absorbed sodium chloride water solution of 0.9 weight percent for 60 minutes, the weight of the superabsorbent polymer after the absorption was determined. The AAP could be obtained by dividing the weight after absorption by the initial weight.

### CS AAP

The determination method of CS AAP was the same as that of the aforementioned AAP, but the time for absorbing the sodium chloride water solution was longer (240 minutes).

### AAP Index

The AAP index was obtained by calculating the ratio value of CS AAP to AAP.

### Residual monomer

The residual monomers of the superabsorbent polymer were measured by the test methods of ERT 410.3(10) by EDANA.

### Absorption rate

The absorption rates of the superabsorbent polymer were measured by the test methods of ERT 420.3(10) by EDANA.

### Gel strength

After 1.000±0.001 g of the superabsorbent polymer was slowly added into 30 mL of the saline solution, followed by stirring with an electromagnetic mixer for 1 minute and standing still for 3 hours or 24 hours, the gel strength of the superabsorbent polymer was determined by STEVENS gel strength analyzer. Noted that the saline solution included 0.005 weight percent of L-ascorbic, and the program of the suspending cylindrical test probe of the STEVENS gel strength texture analyzer was adjusted to a falling speed of 1.0 mm/second and a falling distance of 25 mm.

### Rewet amount

30 sheets of filter paper that the size of each was 8 cm ×20 cm were prepared, and the initial weight (W1) of the 30 sheets of filter paper was measured. Then, 7.8 kg weight with an area of 160 cm² exerted a pressure of 4.8 kPa on the testing absorbent evenly, and 180 mL synthetic urine was dripped in the center of the testing absorbent 3 times with 30-minute intervals. The synthetic urine was the same as that recited in U.S. Patent Application No. 20040106745. Then, the weight on the testing absorbent was removed. 30 sheets of the aforementioned filter paper were placed on testing absorbent, and the weight was placed on the filter paper immediately so that the filter paper could absorb the rewet liquid for 5 minutes. The weight after absorption (W2) between the 30 sheets of filter paper was tested. The difference between the weight after absorption (W2) and the initial weight (W1) of the 30 sheets of filter paper was calculated to obtain the rewet amount of the synthetic urine of the testing absorbent.

### Evaluation result

**Table 1**

| Example | | Preparation Example | | | | | Comparative Preparation Example | | |
|---|---|---|---|---|---|---|---|---|---|
| | | 1 | 2 | 3 | 4 | 5 | 1 | 2 | 3 |
| Collagen | Amount (g) | 15.6 | 156 | 46.8 | 7.8 | 15.6 | 0 | 0.83 | 15.6 |
| | Molecular weight (g/mol) | 1500 | 3000 | 1500 | 1500 | 2300 | 0 | 1500 | 10000 |
| Absorption rate (g/g) | | 59 | 60 | 59 | 62 | 58 | 62 | 62 | 59 |
| Retention capacity (g/g) | | 33.4 | 33.1 | 32.9 | 33.2 | 34.5 | 33.5 | 33.2 | 34.2 |
| AAP (g/g) | | 24.7 | 24.3 | 24.2 | 25.1 | 24.3 | 25.3 | 25.0 | 24.1 |
| CS AAP (g/g) | | 21.0 | 20.9 | 20.5 | 21.6 | 20.1 | 22.1 | 21.7 | 20.2 |
| AAP Indices | | 0.85 | 0.86 | 0.85 | 0.86 | 0.83 | 0.87 | 0.86 | 0.84 |
| Residual monomer (ppm) | | 391 | 413 | 431 | 357 | 471 | 320 | 355 | 477 |
| Gel strength (g) | 3 hours after treated by L-ascorbic | 113 | 127 | 108 | 135 | 107 | 235 | 187 | 201 |
| | 24 hours after treated by L-ascorbic | 52 | 61 | 42 | 72 | 47 | 147 | 115 | 113 |

**Table 2**

| Evaluation items | Preparation Example | | | | | Comparative Preparation Example | | |
|---|---|---|---|---|---|---|---|---|
| | 6 | 7 | 8 | 9 | 10 | 4 | 5 | 6 |
| Basic weight (g/cm³) | 0.08 | 0.09 | 0.08 | 0.08 | 0.07 | 0.08 | 0.08 | 0.07 |
| Thickness (mm) | 16 | 18 | 17 | 16 | 16 | 17 | 16 | 16 |
| Rewet amount (g) | 1.07 | 1.04 | 1.22 | 0.96 | 1.12 | 3.57 | 4.23 | 2.04 |

As shown in Table 1, the superabsorbent polymers of Preparation Example 1 to Preparation Example 5 had an AAP of greater than 15 g/g, AAP indices of bigger than or equal to 0.8, and the rewet amounts of the obtained testing absorbents were smaller than or equal to 3 g, indicating that the superabsorbent polymer still had an excellent absorbent properties with the hydrolyzed collagen added.

Moreover, as shown in Table 1, after being treated with L-ascorbic for 3 hours and 24 hours, compared to that of Comparative Preparation Example 1 to Comparative Preparation Example 3, the gel strengths of the superabsorbent polymers of Preparation Example 6 to Preparation Example 10 were lower indeed, indicating that by adding hydrolyzed collagen of adequate amounts (e.g., greater than 0.83 g) and adequate molecular weight (e.g., 1000 g/mol to smaller than 10000 g/mol), the obtained superabsorbent polymer had better biodegradability.

In addition, compared to the rewet amounts of the testing absorbents of Comparative Preparation Example 4 to Comparative Preparation Example 6, the rewet amounts of the testing absorbents of Preparation Example 6 to Preparation Example 10 were lower, indicating that by adding the hydrolyzed collagen into the superabsorbent polymer, the obtained absorbent still hardly released the absorbed liquid after bearing external stress and thus could keep dryness in use.

Besides, from the evaluation results according to the gel strength according to Table 1, after the superabsorbent polymer was immersed in L-ascorbic for 3 hours or 24 hours, the gel strength significantly decreased and the superabsorbent polymer had an excellent biodegradability.

It can be known from the aforementioned embodiment that the superabsorbent polymer composition of the present invention and the method of manufacturing the superabsorbent polymer have the advantages of adding hydrolyzed collagen so that the obtained superabsorbent polymer has high absorbent properties, high dryness properties, and proper biodegradability. The superabsorbent polymer composition can be applied to personal sanitary products such as diapers, feminine hygiene products, disposable wipes, or incontinence underwear, or can be applied to agricultural or horticultural industries, building industry, petroleum industry, cable line manufacturing industry medical industry as well as entertainment industry.

## Claims

1. A superabsorbent polymer composition, **characterized by** comprising:
a water solution containing carboxylic acid monomers, wherein a neutralization ratio of the water solution containing the carboxylic acid monomers is bigger than or equal to 45 mole percentage, and a pH value of the water solution containing the carboxylic acid monomers is smaller than or equal to 7.0;
hydrolyzed collagen;
a crosslinking agent; and
a polymerization initiator, comprising a thermal decomposition initiator and/or a redox initiator.

2. The superabsorbent polymer composition of claim 1, **characterized by** wherein a molecular weight of the hydrolyzed collagen is bigger than or equal to 1 KDa and smaller than 10 KDa.

3. The superabsorbent polymer composition of claims 1 or 2, **characterized by** wherein based on the superabsorbent polymer composition as 100 weight percent, an amount of the hydrolyzed collagen is 0.1 weight percent to 20 weight percent.

4. The superabsorbent polymer composition of any one of claims 1 to 3, **characterized by** further comprising a surface treatment agent.

5. The superabsorbent polymer composition of any one of claims 1 to 4, **characterized by** wherein the hydrolyzed collagen is obtained by subjecting fish scales to a hydrolyzing step with a protease.

6. The superabsorbent polymer composition of claim 5, **characterized by** wherein the protease is serine endopeptidase.

7. A composite layered structure, **characterized by** comprising:
a non-permeable polyethylene membrane;
an absorbent disposing on the non-permeable polyethylene membrane, wherein the absorbent comprises the superabsorbent polymer composition of claim 1; and
a permeable nonwoven fabric deposed on the absorbent.

8. The composite layered structure of claim 7, **characterized by** wherein the absorbent further comprises pulverized wood pulp mixing with the superabsorbent polymer composition.

9. The composite layered structure of claim 8, **characterized by** wherein based on a weight of the absorbent as 100 weight percent, a weight of the superabsorbent polymer composition is bigger than or equal to 20 weight percent and smaller than 100 weight percent.

10. The composite layered structure of claims 7 or 8, **characterized by** wherein a thickness of the absorbent is not greater than 30 mm.

11. The composite layered structure of any one of claims 7, 8 and 10, **characterized by** wherein a basis weight of the absorbent is 0.01 g/cm² to 0.30 g/cm²

12. A method of manufacturing a superabsorbent polymer, **characterized by** comprising:
performing a neutralizing step on a water solution of carboxylic acid monomers by using a basic compound, so as to obtain a water solution containing carboxylic acid monomers, wherein a neutralization ratio of the water solution containing the carboxylic acid monomers is bigger than or equal to 45 mole percentage, and a pH value of the water solution containing the carboxylic acid monomers is smaller than or equal to 7.0;
mixing the water solution containing the carboxylic acid monomers, hydrolyzed collagen, a crosslinking agent and a polymerization initiator to perform a free radical polymerization reaction, wherein the hydrolyzed collagen is made by subjecting fish scales to a hydrolyzing step with a protease; and
performing a drying step after performing the free radical polymerization reaction to obtain the superabsorbent polymer.

13. The method of manufacturing the superabsorbent polymer of claim 12, **characterized by** further comprising subjecting a surface cross-linking treatment to the superabsorbent polymer.

14. The method of manufacturing the superabsorbent polymer of claims 12 or 13, **characterized by** wherein the protease is serine endopeptidase.

15. The method of manufacturing the superabsorbent polymer of any one of claims 12 to 14, **characterized by** wherein a molecular weight of the hydrolyzed collagen is bigger than or equal to 1 KDa and smaller than 10 KDa.
